# EUROPEAN PATENT SPECIFICATION

(11) **EP 1 896 244 B1**
(45) Date of publication and mention of the grant of the patent: **21.11.2012**
(21) Application number: 06744331.7
(22) Date of filing: 26.05.2006
(51) Int. Cl.: B29C 45/16, B01D 35/30, A61M 16/10, B01D 46/42, B29L 31/14

(54) **IMPROVEMENTS RELATING TO RESPIRATORY APPARATUS**
VERBESSERUNGEN VON BEATMUNGSGERÄTEN
AMELIORATIONS ASSOCIEES A UN APPAREIL RESPIRATOIRE

(30) Priority: 28.05.2005 GB 0511034
(43) Date of publication of application: 12.03.2008
(73) Proprietor: Intersurgical AG, Vaduz (LI)
(72) Inventor: PAYNE, Simon Robert, Goldaming, Surrey GU8 4LU (GB); JASSELL, Surinderjit Kumar, Windsor, Berkshire SL4 5RQ (GB)
(74) Representative: Adamson Jones
(86) International application number: PCT/GB2006/050129
(87) International publication number: WO 2006/129125

(56) References cited:
- EP-A2- 1 430 923
- WO-A2-02/13884
- FR-A1- 2 433 965
- GB-A- 1 224 887
- US-A- 4 986 904
- US-B1- 6 209 541

## Description

This invention relates to respiratory apparatus, and in particular components of a respiratory apparatus comprising a port that requires a releasable closure.

A problem associated with conventional respiratory apparatus is that a port is often provided that requires a releasable closure, and the releasable closure for that port is typically a separate component that is only loosely tethered to the respiratory apparatus. Engagement of the closure with the port may therefore be problematic and time consuming, and there is a risk that the closure will become detached and possibly lost. A detached closure could enter the respiratory system and possibly occlude an exhalation or inhalation passageway.

Document US-B-6209541 discloses a method an a component in accordance with the preambles of claims 1 and 7.

There has now been devised an improved method of manufacturing a component of a respiratory apparatus, and an improved component of a respiratory apparatus, which overcome or substantially mitigate the above-mentioned and/or other disadvantages associated with the prior art.

According to the invention, there is provided a method of manufacturing a component of a respiratory apparatus as recited in Claim 1.

According to a further aspect of the invention, there is provided a component of a respiratory apparatus as recited in Claim 7.

The method of manufacturing a component of a respiratory apparatus, and the component of a respiratory apparatus, according to this aspect of the invention are advantageous because the second material, and hence the closure for the port, is bonded directly to an element of the component. This feature significantly reduces the risk of the closure becoming detached from the component during use, and may also facilitate a user engaging the closure with, and disengaging the closure from, the port.

At least part, and most preferably all, of the first and/or second elements may be translucent so that at least part of the interior of the component is visible during use

The method of manufacturing a component of a respiratory apparatus, and the component of a respiratory apparatus are advantageous because the provision of an opaque element enables the first and second elements to be at least partially translucent, without the need for a separate component or housing for hiding the adhesive used to fix the first and second elements together. Furthermore, the opaque component may be adapted to form a label, which therefore removes the need for a separate label that might become detached during use, and hence also removes the need for an additional assembly process for affixing the label to the component.

In presently preferred embodiments, the second material is injection moulded onto the external surface of one of the elements while that element is incompletely cured, in a so-called two-shot injection moulding process. Alternatively, one of the elements is firstly moulded in a first mould, and then transferred to a second mould, into which the second material is injection moulded onto the external surface of that element when it has completely cured, in a so-called overmoulding injection process. At present, however, a two-shot injection moulding process is preferred because it is a simpler process and hence manufacturing costs are reduced.

The second material is preferably inherently more resilient than the first material. The one or more elements formed of a first material are preferably sufficiently rigid to maintain their shape during normal handling and storage conditions. In presently preferred embodiments, the second material is a thermoplastic elastomer (TPE). For both two-shot injection moulding processes and overmoulding injection processes, the first and second materials are preferably selected so as to form a sufficiently strong and durable bond with each other.

Preferably, the second material includes a connecting arm that projects substantially perpendicularly relative to the external surface of the element to which it is bonded. Most preferably, the connecting arm is sufficiently resilient for it to maintain, substantially, its position relative to the element to which it is bonded despite the action of gravity, but also sufficiently flexible to be deformed such that the closure may be engaged with the port. This feature enables a user to readily locate the closure during use.

The port preferably comprises a gas passageway that projects outwardly from an element of the component and terminates with an exit orifice. The closure preferably has the form of a cap with an end wall that occludes the exit orifice and a side wall that engages the exterior surface of the port with an interference fit, during use. Most preferably, the closure further includes a plug that extends through the exit orifice and engages the interior surface of the port with an interference fit, during use.

The first and/or second elements of the component preferably include raised formations in the form of indicia. The indicia are preferably readily visible to a user, and may therefore take the form of an appropriate label. Most preferably, the opaque element is formed about the raised formations so as to provide a background for the indicia.

In presently preferred embodiments, the raised formations are translucent so that the adhesive is visible through those formations. The adhesive may therefore include a colour pigment that contrasts with the colour of the opaque element so as to improve the visibility of the indicia.

The method according to the invention may be used to manufacture a variety of different types of component for respiratory apparatus. In a particularly preferred embodiment, the method according to the invention is used in the manufacture of a filter. Hence, the one or more elements formed of the first material are preferably formed as one or more housing components that define an enclosure for a filter medium.

A critical factor in the performance of a filter is the quality of the seal between the filter housing and the filter medium. Any imperfections in this seal can dramatically reduce the efficiency of the filter. Hence, in a presently preferred embodiment, the method of manufacturing a filter according to the invention comprises the following steps:
(a) introducing a filter medium into a first housing component, the first housing component including a continuous seat that extends about the periphery of said enclosure;
(b) applying a bead of adhesive to either the seat of the first housing component or a continuous part of the filter medium that is adapted to overlie said seat; steps (a) and (b) being performed in any order; and
(c) engaging the first housing component with a second housing component such that pressure is applied to the bead of adhesive, the housing components being adapted such that application of pressure to the bead of adhesive causes adhesive to be displaced into contact with an adjacent surface of the filter medium and/or an adjacent surface of at least one of the housing components, the adhesive thereby forming a seal between the filter medium and at least one of the housing components.

These method steps are advantageous principally because applying pressure to the bead of adhesive enables the adhesive to penetrate small volumes and hence obtain an improved seal. In particular, this feature enables the adhesive to penetrate any irregularities in the surfaces of the housing components, and also any depressions in the filter medium, without the need to use a large amount of low-viscosity adhesive or reactive expansion adhesive. The method of manufacture according to the invention is therefore less complicated and more cost efficient than prior art methods of manufacture. In addition, the method according to this aspect of the invention may be adapted to bond the first housing component to the second housing component in the same operation as that in which a seal is formed between the filter medium and at least one of the housing components, as discussed in more detail below.

Furthermore, these method steps are advantageous over ultrasonic welding techniques principally because there is a significant reduction in cost, and also improved consistency and quality of the seal between the housing component(s) and the filter medium. There is therefore less risk of leakage of fluid from the filter during use.

The adhesive is preferably a conventional hot melt glue.

The second housing component preferably applies pressure to the bead of adhesive during engagement of the housing components. This application of pressure may be brought about by the second housing component being urged directly against the bead of adhesive, or alternatively by the second housing component being urged against an intermediate component, such as the filter medium, which is consequently urged against the bead of adhesive.

The adhesive is preferably displaced by the application of pressure into a space between overlapping surfaces of the first and second housing components so as to fix those components together. The first and second housing components are therefore preferably dimensioned such that the space between the first and second housing components into which adhesive is displaced is sufficiently large for adhesive to be able to flow into that space, but also sufficiently small so as to minimise the amount of adhesive required.

In presently preferred embodiments, the first and second housing components each have an enclosure-defining wall with an open end that engages with the corresponding open end of the other housing component, during engagement of the housing components. In particular, the enclosure-defining wall of the first housing component preferably has a connection part adjacent to its open end that receives a corresponding connection part of the enclosure-defining wall of the second housing component, most preferably with a relatively close fit, during engagement of the housing components. In this case, the continuous seat of the first housing component is preferably defined on the interior surface of the enclosure-defining wall, immediately adjacent to the connection part.

A space is preferably defined between overlapping surfaces of the connection parts of the first and second housing components, into which adhesive may be displaced during engagement of the housing components so as to fix those components together. Most preferably, the parts of the overlapping surfaces that are immediately adjacent to the continuous seat, when the housing components have been engaged, include formations that define said space. In particular, the interior surface of the connection part of the first housing component and/or the exterior surface of the connection part of the second housing component may include a circumferential recess that defines said space.

The filter medium may have a generally conventional form. For instance, the filter medium may be formed into a web of relatively compressible material, or the filter medium may be formed into a filter block, which is typically substantially rigid in form. Supplementary filter material that is not sealed to a housing component may also be provided, and this supplementary filter material is preferably accommodated within the first and/or second housing components.

Where the filter medium is formed into a web of relatively compressible material, the filter medium may comprise electrostatically-charged polymeric fibres, or any other suitably compressible filter material. In this case, the second housing component preferably clamps the filter medium against the continuous seat of the first housing component, during engagement of the housing components. During manufacture, the filter medium is preferably positioned so that it is supported at its periphery by the continuous seat, and the bead of adhesive is preferably applied to a continuous part of the filter medium that is adapted to overlie said seat, these steps being performed in any order before engagement of the housing components. Most preferably, the bead of adhesive is applied to a surface of the filter medium that faces away from the continuous seat, such that the bead of adhesive is exposed when the filter medium is being supported by said seat. Alternatively, or in addition, a bead of adhesive may be applied to the continuous seat before the filter medium is introduced into the first housing component. Preferably, adhesive is displaced into a space between overlapping surfaces of the housing components, during engagement of the housing components, so as to fix those components together. Furthermore, the end of the second housing component that clamps the filter medium against the continuous seat is preferably provided with formations that define a recess into which adhesive is displaced, during engagement of the housing components, said adhesive acting to form a seal between the filter medium and the end of the second housing component.

Where the filter medium is formed into a filter block, the filter material may comprise sheet material that is pleated and bonded together, or foam material, or a combination of both. The filter block will typically be either generally cuboidal in form, eg having a square cross-section, or generally cylindrical in form, eg having a circular cross-section.

In this case, the first housing component is preferably formed such that the continuous seat is situated adjacent to the side wall of the filter medium once the filter medium has been introduced into the first housing component. The bead of adhesive is preferably applied to the continuous seat either before, or after, the filter medium has been introduced into the first housing component. Most preferably, when pressure is applied to the bead of adhesive, adhesive is not displaced over any part of the ends of the filter medium through which gases enter and exit the filter medium during use. The seat is therefore preferably situated adjacent to the side wall of the filter medium, and substantially equidistant from the ends of the filter medium through which gases enter and exit the filter medium during use.

The filter medium and the first housing component are preferably dimensioned, in this case, such that the space between the filter medium and the first housing component is sufficiently large for adhesive to be able to flow into that space, but also sufficiently small so as to minimise the amount of adhesive required. Preferably, the enclosure-defining wall of the first housing component has a holding part of reduced cross-sectional dimensions relative to the connection part. The filter medium is preferably received with a relatively close fit within the holding part, but a space is preferably defined between the interior surface of the connection part and the filter medium. In this case, the seat preferably takes the form of a shoulder between the holding part and the connection part of the first housing component. The enclosure-defining wall of the second housing component preferably either abuts the seat, or has a separation from the seat that is filled with adhesive, when the first and second housing components are fully engaged. Most preferably, the interior surface of the enclosure-defining wall of the second housing component aligns with the interior surface of the holding part of the first housing component. In this case, the filter medium is preferably accommodated within the enclosure defined by the first and second housing components with a relatively close fit.

Regardless of the form of the filter medium, each housing component preferably comprises at least one port that enables the inflow and/or outflow of gas during normal use. The filter is preferably adapted for inclusion within an artificial respiratory circuit, and most preferably each of the ports has the form of a standard respiratory apparatus connector.

Preferred embodiments of the invention will now be described in greater detail, by way of illustration only, with reference to the accompanying drawings, in which
Figure 1 is a series of cross-sectional views showing three stages of a method of manufacturing a filter according to the invention;
Figure 2 is a perspective view of a first embodiment of a filter according to the invention;
Figure 3 is a view similar to that of Figure 2, but partly cut-away;
Figure 4 is a perspective view of a second embodiment of a filter according to the invention;
Figure 5 is a view that is similar to that of Figure 4, but partly cut-away;
Figure 6 is a fragmentary view of the filter of Figures 4 and 5; and
Figure 7 is a perspective view of a third embodiment of a filter according to the invention.

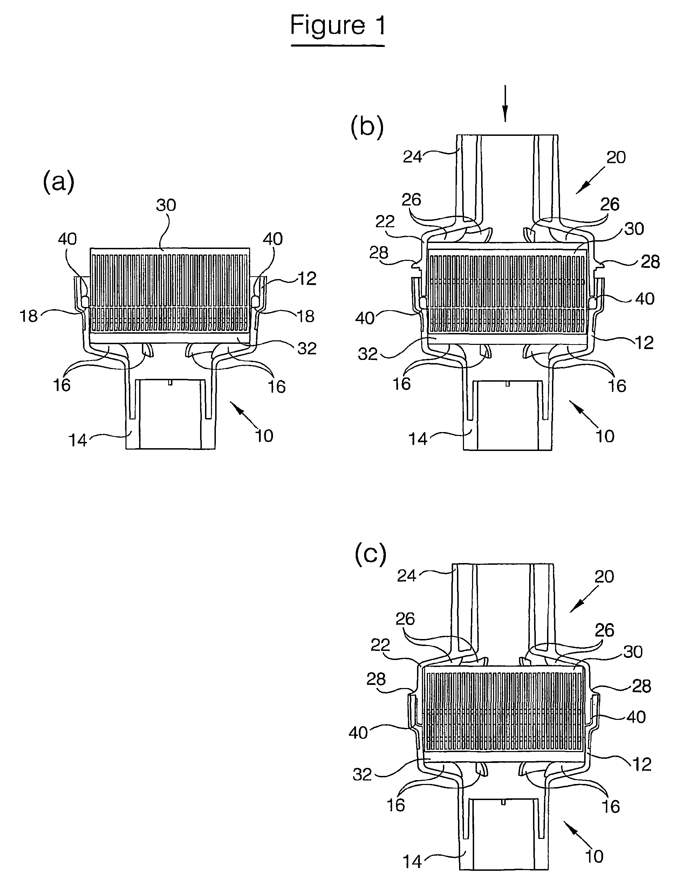

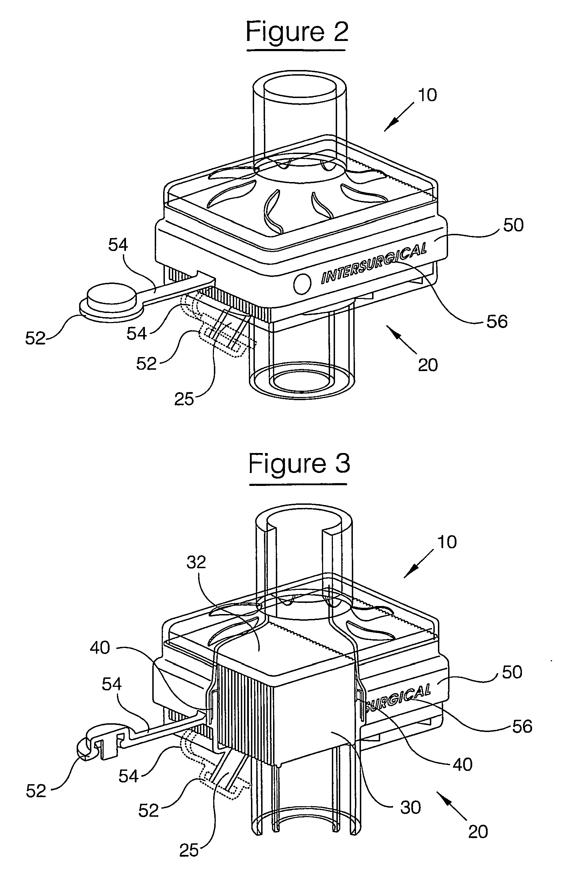

Three stages of a method of manufacturing a filter according to the invention are shown in Figure 1, and a first embodiment of a filter according to the invention that has been manufactured by that method is shown in Figures 2 and 3. However, before these stages of the method are carried out, a pair of cooperating housing components 10,20, and suitable filter media 30,32 are manufactured or obtained.

The filter media 30,32 may be conventional. In particular, the filter media 30 shown in Figures 1 to 3 comprises a first filter media 30 having a pleated structure and a second, relatively smaller, filter medium 32 having a foam-like structure. The first filter medium 30 and the second filter medium 32 are not bonded together, but are held alongside one another in the assembled filter. The second filter medium 32 may be a fibre capturing scrim or an HME element.

The first housing component 10 comprises an enclosure-defining wall 12 and a cylindrical connector 14 extending therefrom. The enclosure-defining wall 12 has one open end, and one end from which the connector 14 extends. The connector 14 has the form of a port that enables the inflow and outflow of gas during normal use, and is adapted to connect to standard respiratory apparatus. The connector 14 has a smaller diameter than the enclosure-defining wall 12 such that a shoulder is formed on the interior surface of the first housing component 10 between these two walls 12,14. A series of upstanding ribs 16 are provided on this shoulder so as to space the second filter medium 32 from this shoulder in the assembled filter, and hence ensure that gases flow through the entire filter media 30,32 during use. In addition, the enclosure-defining wall 12 has a connection part adjacent to its open end that has increased internal dimensions relative to the remainder of the enclosure-defining wall 12, such that a continuous shoulder 18 is defined on the interior surface of the enclosure-defining wall 12.

An elastomeric band 50 is formed over the exterior surface of the enclosure-defining wall 12 of the first housing component 10, and includes an integral closure 52 and connecting arm 54, as shown in Figures 2 and 3. The first housing component 10 and elastomeric band 50 are formed together using a so-called two-shot injection moulding process. In particular, the first housing component 10 is injection moulded in a suitably strong and relatively rigid plastics material, such as polypropylene, and then a thermoplastic elastomer (TPE) is injection moulded onto the external surface of the first housing component 10, while it is incompletely cured, so as to form the elastomeric band 50. Since the elastomeric band 50 is moulded directly onto the first housing component 10, these components 10,50 become bonded together.

The second housing component 20 is similar to the first housing component 10, and comprises an enclosure-defining wall 22, a cylindrical connector 24 extending therefrom, and a series of upstanding ribs 26 on the shoulder that is formed on the interior surface of the housing component 20 between these two walls 22,24. The second housing component 20 is injection moulded in a suitably strong and relatively rigid plastics material, such as polypropylene.

In contrast to the first housing component 10, the enclosure-defining wall 22 has a connection part adjacent to its open end that has internal cross-sectional dimensions that are equal to those of the remainder of the enclosure-defining wall 22, such that it fits relatively closely within the connection part of the enclosure-defining wall 12 of the first housing component 10. A small space, however, exists between the connection parts of the first and second housing components 10,20 when the components 10,20 are so engaged. A flange 28 is also provided on the exterior surface of the enclosure-defining wall 22 that is adapted to abut the end of the first housing component 10 when the connection part of the second housing component 20 has been fully received within the connection part of the first housing component 10. In this way, the housing components 10,20 are engaged during manufacture so as to define an enclosure for the filter media 30,32.

In addition, the second housing component 20 is provided with a sampling port 25 (shown in Figures 2 and 3) that enables gases to be extracted from the interior of the enclosure-defining wall 22 of the second housing component 20, during use, for testing. The sampling port 25 is cylindrical in form, and extends outwardly from an opening in the shoulder formed between the enclosure-defining wall 22 and the connector wall 24 of the second housing component 20.

The connecting arm 54 of the elastomeric band 50 extends perpendicularly from the external surface of the first housing component 10, and is adapted to deform such that the closure 52 can be brought into engagement with the sampling port 25, as illustrated in Figures 2 and 3. The closure 52 comprises an end wall for occluding the exit orifice of the sampling port 25, a cylindrical side wall for engaging the exterior surface of the sampling port 25 with an interference fit, and a plug for engaging the interior surface of the sampling port 25 with an interference fit.

Once the first housing component 10, second housing component 20, and filter media 30,32, have been manufactured or obtained, these components 10,20,30,32 are then assembled together as shown in Figure 1, which shows three stages of the method of manufacturing a filter according to the invention. Firstly, the second filter medium 32 is inserted into the enclosure-defining wall 12 of the first housing component 10 with a relatively close fit, such that the second filter medium 32 rests upon the ribs 16 of the first housing component 10. The first filter medium 30 is then inserted into the enclosure-defining wall 12 of the first housing component 10 with a relatively close fit, such that the first filter medium 30 rests upon the exposed surface of the second filter medium 32, and an upper part of the first filter medium 30 projects from the open end of the enclosure-defining wall 12. A small space, however, exists between the side wall of the filter medium 30 and the interior surface of the first housing component 10. A continuous bead of adhesive 40, which is a conventional hot melt glue, is then applied to the shoulder 18 on the internal surface of the enclosure-defining wall 12. This arrangement is shown in Figure 1 a.

The second housing component 20 is then brought into engagement with the first housing component 10, whilst the adhesive 40 is still molten, such that the part of the filter medium 30 that projects from the first housing component 10 is received within the enclosure-defining wall 22. In particular, the connection part of the enclosure-defining wall 22 of the second housing component 20 is received with a relatively close fit, as discussed above, within the connection part of the enclosure-defining wall 12 of the first housing component 10, as shown in Figure 1b.

As the second housing component 20 is brought into engagement with the first housing component 10, the end surface of the second housing component 20 will come into contact with, and apply pressure to, the bead of adhesive 40 on the shoulder 18 of the first housing component 10. The first and second housing components 10,20 are brought together under sufficient pressure for the bead of adhesive 40 to be compressed by the end surface of the second housing component 20. This action is continued until the flange 28 of the second housing component 20 is brought into abutment with the end of the first housing component 10, as shown in Figure 1 c.

As shown in Figure 1c, compression of the bead of adhesive 40 causes the adhesive 40 to flow into the small space that exists between the connection parts of the first and second housing components 10,20, and also the small space that exists between the side wall of the filter medium 30 and the interior surface of the first and second housing components 10,20. The adhesive 40 situated between the connection parts of the first and second housing components 10,20 acts to fix these components 10,20 together, and the adhesive 40 situated between the side wall of the filter medium 30 and the interior surface of the first and second housing components 10,20 acts to form a seal about the periphery of the filter medium 30.

The application of pressure to the bead of adhesive 40 enables the adhesive to penetrate any irregularities in the surfaces being sealed, and also any depressions in the filter medium 30, so that an improved seal is obtained. The need to use a large amount of low-viscosity adhesive or reactive expansion adhesive is also removed, such that this method of manufacture is less complicated and more cost efficient than prior art methods of manufacture. Furthermore, since the adhesive 40 is situated between the side wall of the filter medium 30 and the interior surface of the first and second housing components 10,20, the adhesive 40 does not extend over an end surface of the filter medium 30 and hence does not restrict the surface area of the filter medium 30 through which gases are able to pass during use.

As shown in Figures 2 and 3, the first and second housing components 10,20 are translucent, and the elastomeric band 50 is opaque. Since the elastomeric band 50 extends over the external surface of the connection part of the first housing component 10, the irregular band of adhesive 40 that is formed between the connection parts of the first and second housing components 10,20 is hidden from the user by the elastomeric band 50.

In addition, the exterior surface of the first housing component 10 is formed with raised formations 56 having the form of indicia. The elastomeric band 50 is moulded onto the exterior surface of the connection part of the first housing component 10 such that an outer surface of each raised formation 56 is exposed and is aligned flush with the exterior surface of the elastomeric band 50. The elastomeric band 50 forms a background for the indicia, such that the indicia are readily visible to a user. This feature removes the need for a separate label that might become detached during use, and hence also removes the need for an additional assembly process for affixing the label to the filter housing.

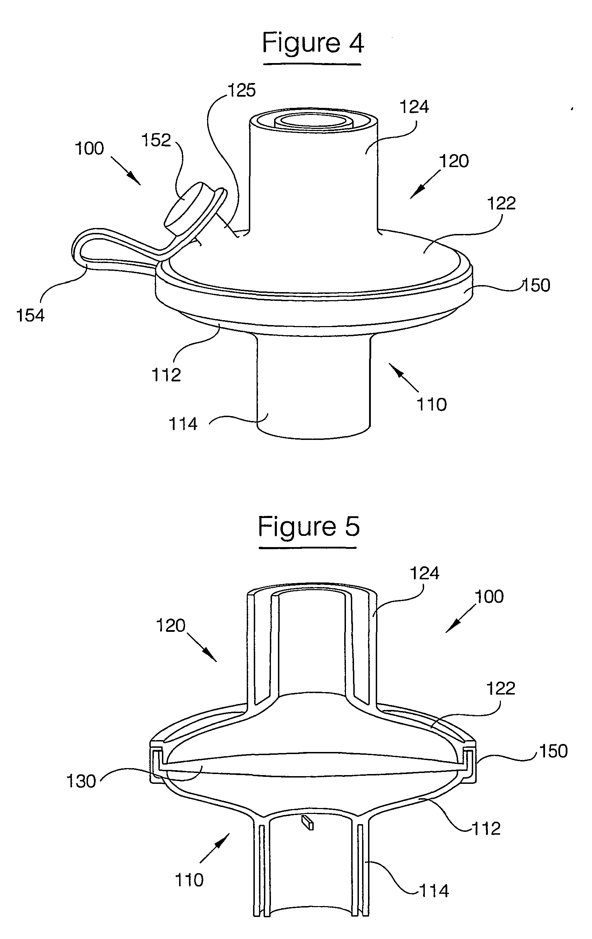

Figures 4 to 6 show a second embodiment of a filter according to the invention, which is generally designated 100. The filter 100 comprises a pair of cooperating housing components 110,120, and a filter medium 130.

The first housing component 110 comprises an enclosure-defining wall 112 and a cylindrical connector 114 extending therefrom. The enclosure-defining wall 112 has one open end, and one end from which the connector 114 extends. The connector 114 has the form of a port that enables the inflow and outflow of gas during normal use, and is adapted to connect to standard respiratory apparatus.

The enclosure-defining wall 112 has a connection part adjacent to its open end that has increased internal cross-sectional dimensions relative to the remainder of the enclosure-defining wall 112, such that a continuous shoulder 118 is defined on the interior surface of the enclosure-defining wall 112. In addition, an elastomeric band 150 is formed over the exterior surface of the enclosure-defining wall 112 of the first housing component 110, and includes an integral closure 152 and connecting arm 154, as shown in Figure 4. The first housing component 110 and elastomeric band 150 are formed together using a so-called two-shot injection moulding process, as discussed above in relation to the first example of a filter manufactured by the method according to the invention.

The second housing component 120 is similar to the first housing component 110, and comprises an enclosure-defining wall 122, and a cylindrical connector 124 extending therefrom. The enclosure-defining wall 122 has a connection part adjacent to its open end that fits relatively closely within the connection part of the enclosure-defining wall 112 of the first housing component 110. A flange 128 is also provided on the exterior surface of the enclosure-defining wall 122 that is adapted to abut the end of the first housing component 110 when the connection part of the second housing component 120 has been fully received within the connection part of the first housing component 110. In this way, the housing components 110,120 are engaged during manufacture so as to define an enclosure for the filter medium 130.

However, the connection part of the enclosure-defining wall 122 of the second housing component 120 includes a circumferential recess on its exterior surface. In particular, the recess extends from a position a short distance below the flange 128, to the end of the connection part of the second housing component 120. In this way, a small circumferential space exists between the connection parts of the first and second housing components 110,120, in a region that is adjacent to the end of the connection part the second housing component 120, when the components 110,120 are so engaged. The circumferential recess also includes a step so that the recess has a portion of greater depth at the end of the connection part of the second housing component 120.

In addition, the second housing component 120 is provided with a sampling port 125, and associated closure 152 with connecting arm 154, that are essentially identical to those of the first embodiment of a filter according to the invention, as discussed above.

The filter medium 130 is a cylindrical web of unwoven polymeric fibres that has a diameter substantially equal to that of the interior surface of the connection part of the first housing component 110. In particular, the filter medium 130 is adapted to be received within the connection part of the first housing component 110 with a relatively close fit.

Once the first housing component 110, second housing component 120, and filter medium 130, have been manufactured or obtained, these components 110,120,130 are then assembled together using a method that is similar to that shown in Figure 1. However, the method of assembling this filter 100 differs in a number of respects.

In particular, the filter medium 130 is firstly located within the first housing component 110 such that it is supported at its periphery by the continuous shoulder 130 of that component 110. A continuous bead of adhesive 140, which is a conventional hot melt glue, is then applied to the peripheral portion of the filter medium 140 that is situated above the continuous shoulder 118 on the internal surface of the enclosure-defining wall 112.

The second housing component 120 is then brought into engagement with the first housing component 110, whilst the adhesive 140 is still molten. In particular, the connection part of the enclosure-defining wall 122 of the second housing component 120 is received with a relatively close fit, as discussed above, within the connection part of the enclosure-defining wall 112 of the first housing component 110.

As the second housing component 120 is brought into engagement with the first housing component 110, the end surface of the second housing component 120 will come into contact with, and apply pressure to, the bead of adhesive 140 on the peripheral portion of the filter medium 140 that is situated above the continuous shoulder 118 of the first housing component 110. The first and second housing components 110,120 are brought together under sufficient pressure for the bead of adhesive 140, and also the filter medium 140, to be compressed by the end surface of the second housing component 120. This action is continued until the flange 128 of the second housing component 20 is brought into abutment with the end of the first housing component 110.

Compression of the bead of adhesive 140 causes the filter medium 140 to become clamped between the first and second housing components 110,120, and also causes the adhesive 140 to flow into the small circumferential space that exists between the connection parts of the first and second housing components 110,120, as discussed above. The adhesive 140 situated between the connection parts of the first and second housing components 110,120 acts to fix these components 110,120 together, and the adhesive 140 situated between the filter medium 30 and the second housing component 120 acts to form a seal about the periphery of the filter medium 130.

The application of pressure to the bead of adhesive 140 enables the adhesive to penetrate any irregularities in the surfaces being sealed, and also any depressions in the filter medium 130, so that an improved seal is obtained. Furthermore, this method of manufacture achieves improved consistency and quality of seal between the housing components 110,120 and the filter medium 130 relative to ultrasonic welding techniques. There is therefore less risk of leakage of fluid from the filter during use

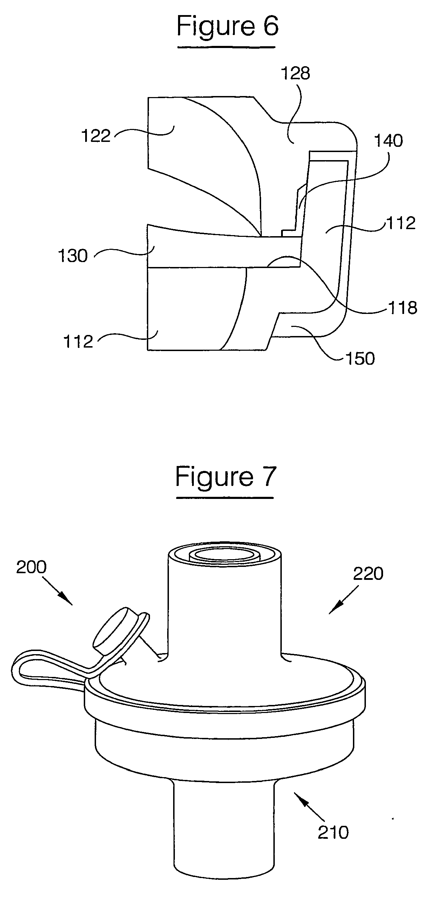

Finally, Figure 7 shows a third embodiment of a filter according to the invention, which is generally designated 200. This filter 200 has a structure and method of manufacture that is identical to the first example discussed above, save for the housing components 210,220 having a circular, rather than square, cross-sectional shape, and the filter media including a HME element. The HME element acts to restrict the escape of heat and moisture from the respiratory system during use.

## Claims

1. A method of manufacturing a component of a respiratory apparatus including a port (25, 125) that enables access to gas within the component, during use, the method comprising the steps of:
(a) moulding first (10, 110) second (20, 120) elements of the component in a first, plastics material, at least one of which elements includes the access port (25, 125) of the component;
(b) fixing the first (10, 110) and second (20, 120) elements together with adhesives (40, 140); and
(c) injection moulding a second, elastomeric material onto an external surface of one of the elements (10, 110, 20, 120),
**characterised in that** the second material defines an opaque element (50, 150) on the external surface of the first (10, 110) or second (20, 120) element which hides the adhesive (40, 140) of the the assembled component, the second material including an integral closure (52, 152) for the port (25, 125) and a connecting arm (54, 154) that projects outwardly relative to the external surface of the element to which it is bonded; steps (b) and (c) being performed in any order.

2. A method as claimed in Claim 1, wherein the first (10, 110) and second (20, 120) elements comprise relatively rigid elements and the second material is injection moulded onto the external surface of one of the relatively rigid elements while that element is incompletely cured.

3. A method as claimed in Claim 1, wherein one of the elements (10, 110, 20, 120) is firstly moulded in a first mould, and then transferred to a second mould, into which the second material is injection moulded onto the external surface of that element when it has completely cured.

4. A method as claimed in Claim 1, wherein at least part of the first and/or second elements (10, 110, 20, 120) are translucent so that at least part of the interior of the component is visible during use.

5. A method as claimed in Claim 4, wherein the first and/or second element (10, 110, 20, 120) of the component include raised formations (56) in the form of indicia, and the opaque element (50, 150) is formed about the raised formations (56) so as to provide a background for the indicia.

6. A method as claimed in any preceding claim, wherein the first and second elements (10, 110, 20, 120) formed of the first material are formed as one or more housing components that define an enclosure for a filter medium (30, 130).

7. A component of a respiratory apparatus comprising:
first (10, 110) and second (20, 120) elements formed of a first, plastics material, the first and second elements being fixed together by adhesive (40, 140), at least one of which elements includes a port (25, 125) that enables access to gas within the component, during use; and
a second, elastomeric material bonded to an external surface of the first (10, 110) or second (20, 120) element,
**characterised in that** the second material defines an opaque element (50, 150) on the external surface of the first (10, 110) or second (20, 120) element which hides the adhesive (40, 140) of the assembled component, the second material including an integral closure (52, 152) for the port (25, 125) and a connecting arm (54, 154) that projects outwardly relative to the external surface of the element to which it is bonded.

8. A component of a respiratory apparatus as claimed in Claim 7, wherein the first (10, 110) and (20, 120) elements comprise relatively rigid elements and the connecting arm (54, 154) sufficiently resilient for it to maintain, substantially, its position relative to the relatively rigid element to which it is bonded despite the action of gravity, but also sufficiently flexible to be deformed such that the closure (52, 152) may be engaged with the port (25 125).

9. A component of a respiratory apparatus as claimed in Claim 7 or Claim 8, wherein the port (25, 125) comprises a gas passageway that projects outwardly from the first (10, 110) or second (20, 120) element and terminates with an exit orifice.

10. A component of a respiratory apparatus as claimed in Claim 9, wherein the closure (52, 152) has the form of a cap with an end wall that occludes the exit orifice and a side wall that engages the exterior surface of the port with an interference fit, during use.

11. A component of a respiratory apparatus as claimed in Claim 10, wherein the closure (52, 152) further includes a plug that extends through the exit orifice and engages the interior surface of the port with an interference fit, during use.

12. A component of a respiratory apparatus as claimed in Claim 7, wherein at least part of the first and/or second elements (10, 110, 20, 120) are translucent so that at least part of the interior of the component is visible during use.

13. A component of a respiratory apparatus as claimed in any one of Claims 7 to 12, wherein the component has the form of a filter, the first and second elements (10, 110, 20, 120) formed of the first material being one or more housing components that define an enclosure for a filter medium (30, 130).

## Patentansprüche

1. Verfahren zur Herstellung einer Komponente eines Atemgeräts mit einem Anschluss (25, 125), der beim Gebrauch Zugang zu Gas in der Komponente gewährt, wobei das Verfahren die folgenden Schritte beinhaltet:
(a) Formen eines ersten (10, 110) und zweiten (20, 120) Elements der Komponente aus einem ersten Kunststoffmaterial, wobei wenigstens eines der Elemente den Zugangsanschluss (25, 125) der Komponente beinhaltet;
(b) Befestigen des ersten (10, 110) und zweiten (20, 120) Elements mit einem Klebstoff (40, 140) aneinander; und
(c) Spritzformen eines zweiten elastomeren Materials auf eine Außenseite von einem der Elemente (10, 110, 20, 120),
**dadurch gekennzeichnet, dass** das zweite Material ein opakes Element (50, 150) auf der Außenseite des ersten (10, 110) oder zweiten (20, 120) Elements definiert, das den Klebstoff (40, 140) der zusammengefügten Komponente verbirgt, wobei das zweite Material ein integriertes Gehäuse (52, 152) für den Anschluss (25, 125) und einen Verbindungsarm (54, 154) beinhaltet, der relativ zur Außenseite des Elements, auf das er geklebt wurde, nach außen vorsteht; wobei die Schritte (b) und (c) in einer beliebigen Reihenfolge ausgeführt werden.

2. Verfahren nach Anspruch 1, wobei das erste (10, 110) und zweite (20, 120) Element relativ starre Elemente umfassen und das zweite Material auf die Außenseite von einem der relativ starren Elemente spritzgeformt wird, während dieses Element unvollständig gehärtet ist.

3. Verfahren nach Anspruch 1, wobei eines der Elemente (10, 110, 20, 120) zunächst in einer ersten Form geformt und dann in eine zweite Form übertragen wird, in die das zweite Material auf die Außenseite dieses Elements spritzgeformt wird, wenn es vollständig ausgehärtet ist.

4. Verfahren nach Anspruch 1, wobei wenigstens ein Teil des ersten und/oder zweiten Elements (10, 110, 20, 120) lichtdurchlässig ist, so dass wenigstens ein Teil des Inneren der Komponente beim Gebrauch sichtbar ist.

5. Verfahren nach Anspruch 4, wobei das erste und/oder zweite Element (10, 110, 20, 120) der Komponente erhabene Formationen (56) in Form von Zeichen aufweist/-en und das opake Element (50, 150) um die erhabenen Formationen (56) herum ausgebildet ist, um einen Hintergrund für die Zeichen zu geben.

6. Verfahren nach einem der vorherigen Ansprüche, wobei das aus dem ersten Material gebildete erste und zweite Element (10, 110, 20, 120) als eine oder mehrere Gehäusekomponenten gebildet sind, die ein Gehäuse für ein Filtermedium (30, 130) definieren.

7. Komponente eines Atemgeräts, die Folgendes umfasst:
ein aus einem ersten Kunststoffmaterial gebildetes erstes (10, 110) und zweites (20, 120) Element, wobei das erste und zweite Element mit Klebstoff (40, 140) aneinander befestigt sind, wobei wenigstens eines der Elemente einen Anschluss (25, 125) aufweist, der beim Gebrauch Zugang zu Gas in der Komponente gewährt; und
ein zweites elastomeres Material, das auf eine Außenseite des ersten (10, 110) oder zweiten (20, 120) Elements geklebt ist,
**dadurch gekennzeichnet, dass** das zweite Material ein opakes Element (50, 150) auf der Außenseite des ersten (10, 110) oder zweiten (20, 120) Elements definiert, das den Klebstoff (40, 140) der zusammengefügten Komponente verbirgt, wobei das zweite Material ein integriertes Gehäuse (52, 152) für den Anschluss (25, 125) und einen Verbindungsarm (54, 154) beinhaltet, der relativ zur Außenseite des Elements, auf das er geklebt ist, nach außen vorsteht.

8. Komponente eines Atemgeräts nach Anspruch 7, wobei das erste (10, 110) und zweite (20, 120) Element relativ starre Elemente umfassen und der Verbindungsarm (54, 154) elastisch genug ist, um seine Position relativ zu dem relativ starren Element, auf das er geklebt ist, trotz Schwerkraftwirkung im Wesentlichen zu halten, aber flexibel genug ist, um sich zu verformen, so dass das Gehäuse (52, 152) mit dem Anschluss (25, 125) in Eingriff kommen kann.

9. Komponente eines Atemgeräts nach Anspruch 7 oder 8, wobei der Anschluss (25, 125) einen Gaskanal umfasst, der vom ersten (10, 110) oder zweiten (20, 120) Element nach außen vorsteht und mit einer Austrittsöffnung endet.

10. Komponente eines Atemgeräts nach Anspruch 9, wobei das Gehäuse (52, 152) die Form einer Kappe mit einer die Austrittsöffnung verschließenden Endwand und einer Seitenwand hat, die beim Gebrauch mit Presspassung in die Außenseite des Anschlusses eingreift.

11. Komponente eines Atemgeräts nach Anspruch 10, wobei das Gehäuse (52, 152) ferner einen Stopfen beinhaltet, der durch die Austrittsöffnung verläuft und beim Gebrauch mit Presspassung in die Außenseite des Anschlusses eingreift.

12. Komponente eines Atemgeräts nach Anspruch 7, wobei wenigstens ein Teil des ersten und/oder zweiten Elements (10, 110, 20, 120) lichtdurchlässig ist, so dass beim Gebrauch wenigstens ein Teil des Inneren der Komponente sichtbar ist.

13. Komponente eines Atemgeräts nach einem der Ansprüche 7 bis 12, wobei die Komponente die Form eines Filters hat, wobei das erste und zweite aus dem ersten Material gebildete Element (10, 110, 20, 120) ein oder mehrere Gehäuseelemente sind, die ein Gehäuse für ein Filtermedium (30, 130) definieren.

## Revendications

1. Procédé de fabrication d'un composant d'un appareil respiratoire comprenant un port (25, 125) qui permet d'accéder à un gaz à l'intérieur du composant, en cours d'utilisation, le procédé comprenant les opérations suivantes :
(a) le moulage d'un premier (10, 110) et d'un deuxième (20, 120) éléments du composant dans un premier matériau plastique, au moins l'un desdits éléments comprenant le port d'accès (25, 125) du composant,
(b) le rattachement du premier (10, 110) et du deuxième (20, 120) éléments l'un à l'autre avec un adhésif (40, 140), et
(c) le moulage par injection d'un deuxième matériau élastomère sur une surface externe de l'un des éléments (10, 110, 20, 120) **caractérisé en ce que** le deuxième matériau définit un élément opaque (50, 150) sur la surface externe du premier (10, 110) ou du deuxième (20, 120) élément qui masque l'adhésif (40, 140) du composant assemblé, le deuxième matériau comprenant une fermeture intégrée (52, 152) pour le port (25, 125) et un bras de connexion (54, 154) qui se projette vers l'extérieur par rapport à la surface externe de l'élément auquel il est lié, les opérations (b) et (c) étant exécutées dans un ordre quelconque.

2. Procédé selon la Revendication 1, où les premier (10, 110) et deuxième (20, 120) éléments comprennent des éléments relativement rigides et le deuxième matériau est moulé par injection sur la surface externe de l'un des éléments relativement rigides, cet élément étant incomplètement durci.

3. Procédé selon la Revendication 1, où un des éléments (10, 110, 20, 120) est d'abord moulé dans un premier moule et ensuite transféré vers un deuxième moule, dans lequel le deuxième matériau est moulé par injection sur la surface externe cet élément lorsqu'il a complètement durci.

4. Procédé selon la Revendication 1, où au moins une partie du premier et/ou du deuxième élément (10, 110, 20, 120) est translucide de sorte qu'au moins une partie de l'intérieur du composant soit visible en cours d'utilisation.

5. Procédé selon la Revendication 4, où le premier et/ou le deuxième élément (10, 110, 20, 120) du composant comprend des protubérances (56) sous la forme de repères, et l'élément opaque (50, 150) est formé autour des protubérances (56) de façon à fournir un arrière-plan aux repères.

6. Procédé selon l'une quelconque des Revendications précédentes, où les premier et deuxième éléments (10, 110, 20, 120) formés à partir du premier matériau sont formés sous la forme d'un ou plusieurs composants de boîtier qui définissent un logement pour un milieu de filtrage (30, 130).

7. Composant d'un appareil respiratoire comprenant :
un premier (10, 110) et un deuxième (20, 120) éléments formés à partir d'un premier matériau plastique, les premier et deuxième éléments étant fixés l'un à l'autre par un adhésif (40, 140), au moins l'un desdits éléments comprenant un port (25, 125) qui permet d'accéder à un gaz à l'intérieur du composant, en cours d'utilisation, et
un deuxième matériau élastomère lié à une surface externe du premier (10, 110) ou du deuxième (20, 120) élément,
**caractérisé en ce que** le deuxième matériau définit un élément opaque (50, 150) sur la surface externe du premier (10, 110) ou du deuxième (20, 120) élément qui masque l'adhésif (40, 140) du composant assemblé, le deuxième matériau comprenant une fermeture intégrée (52, 152) pour le port (25, 125) et un bras de connexion (54, 154) qui se projette vers l'extérieur par rapport à la surface externe de l'élément auquel il est lié.

8. Composant d'un appareil respiratoire selon la Revendication 7, où les premier (10, 110) et deuxième (20, 120) éléments comprennent des éléments relativement rigides et le bras de connexion (54, 154) est suffisamment élastique pour qu'il maintienne sensiblement sa position par rapport à l'élément relativement rigide auquel il est lié en dépit de l'action de la gravité, mais également suffisamment souple pour être déformé de sorte que la fermeture (52, 152) puisse entrer en prise avec le port (25, 125).

9. Composant d'un appareil respiratoire selon la Revendication 7 ou 8, où le port (25, 125) comprend une voie de passage de gaz qui se projette vers l'extérieur à partir des premier (10, 110) ou deuxième (20, 120) éléments et se termine par un orifice de sortie.

10. Composant d'un appareil respiratoire selon la Revendication 9, où la fermeture (52, 152) a la forme d'un capuchon avec une paroi d'extrémité qui bouche l'orifice de sortie et une paroi latérale qui entre en prise avec la surface extérieure du port dans un ajustement serré en cours d'utilisation.

11. Composant d'un appareil respiratoire selon la Revendication 10, où la fermeture (52, 152) comprend en outre un bouchon qui s'étend au travers de l'orifice de sortie et entre en prise avec la surface intérieure du port dans un ajustement serré en cours d'utilisation.

12. Composant d'un appareil respiratoire selon la Revendication 7, où au moins une partie des premier et/ou deuxième éléments (10, 110, 20, 120) est translucide de sorte qu'au moins une partie de l'intérieur du composant soit visible en cours d'utilisation.

13. Composant d'un appareil respiratoire selon l'une quelconque des Revendications 7 à 12, où le composant a la forme d'un filtre, les premier et deuxième éléments (10, 110, 20, 120) formés à partir du premier matériau étant un ou plusieurs composants de boîtier qui définissent un logement pour un milieu de filtrage (30, 130).
